# EUROPÄISCHE PATENTANMELDUNG

(11) **EP 1 510 226 A2**
(43) Veröffentlichungstag der Anmeldung: **02.03.2005**
(21) Anmeldenummer: 04405315.5
(22) Anmeldetag: 21.05.2004
(51) Int. Cl.: A61L 2/03, A61L 2/02, A61L 2/00, B01J 19/08

(54) **Verfahren und Vorrichtung zum Sterilisieren der Oberflächen von Gegenständen wie chirurgische Instrumente, Prothesen oder Aehnlichem**

(30) Priorität: 18.08.2003 CH 141003
(71) Anmelder: Mont.El. S.r.l., 25057 Sale Marasino (BS) (IT)
(72) Erfinder: Veschi, Raffaele, 01408-020 San Paolo (BR)
(74) Vertreter: Gaggini, Carlo

(57) **Zusammenfassung**

Ein Verfahren zur Oberflächen-Sterilisation von Gegenständen und zur Entfernung von Bakterienfilmen mittels eines magnetischen und/oder elektrischen Feldes sowie die entsprechenden Vorrichtungen werden beschrieben.

## Beschreibung

Die vorliegende Erfindung betrifft ein Verfahren und entsprechende Vorrichtungen zum Sterilisieren der Oberfläche von Gegenständen wie chirurgischen Instrumente, Prothesen oder ähnlichen Gegenständen. Insbesondere erlauben die Verfahren und die Vorrichtungen gemäss der vorliegenden Erfindung, auf einer grossen Vielfalt von Oberflächen, beispielsweise von chirurgischen Instrumenten, Kontaktlinsen, Prothesen, oder ähnlichen Gegenständen, die Bildung eines Bakterienfilms zu verhindern und einen solchen zu entfernen.

Die Anwendung des Verfahrens gemäss der vorliegenden Erfindung richtet sich auf die betreffenden Oberflächen, die mit einem über die Zeit veränderlichen elektrischen Feld und/oder einem magnetischen Feld beaufschlagt werden, dessen Eigenschaften in Funktion der Abmessungen der Zone reguliert werden können, in welche die zu behandelnde Oberfläche eingebracht wird, in solcher Weise, dass allenfalls vorhandene Bakterien und Biofilme entfernt werden.

Auf diese Weise gelingt es, Bakterienfilme zu behandeln und zu entfernen, die gegen andere Systeme, wie beispielsweise Antibiotika oder ähnliche, resistent geworden sind, wobei ein wirksamer Sterilisations-Effekt auch in jenen Fällen erreicht wird, in denen die bekannten Techniken nicht angewendet werden können oder wenig wirksame Resultate ergeben.

Die vorliegenden Erfindung betrifft auch die Vorrichtungen zur Ausübung des genannten Verfahren.

In zahlreichen Situationen ist einfaches und wirtschaftliches Sterilisieren von kleinen Gegenständen notwendig, beispielsweise von chirurgischen Instrumenten und Werkzeugen, Prothesen und dergleichen.

Viele Verfahren mit dieser Zielsetzung sind bekannt, die von der Anwendung von Lösungen, die Desinfektionsmittel enthalten, bis zur Behandlung mit Luft oder Flüssigkeiten bei hoher Temperatur (120-150°C), oder zur Anwendung von Ultraviolettstrahlung reichen.

Ebenfalls bekannt sind Vorrichtungen, die elektromagnetische Felder anwenden, um Gegenstände zu sterilisieren, wie beispielsweise in den Patenten USA 5'632'955, 5'326'530, 3'753'651 und WO 01/02023 beschrieben.

Dabei handelt es sich jedoch um Vorrichtungen der Art von Mikrowellengeneratoren, die darauf ausgerichtet sind, den ganzen Gegenstand zu sterilisieren, wobei dieser durchgehend aufgeheizt wird.

Solche Vorrichtungen erweisen sich als ungeeignet zum Erreichen der Oberflächen-Sterilisation von Gegenständen, und auch die andern Systeme erweisen sich nicht immer als für diesen Zweck geeignet.

Wenn Gegenstände zu sterilisieren sind, die Teile aus Kunststoff enthalten, wie beispielsweise Zahnprothesen, Kontaktlinsen oder Ähnliches, ist die Anwendung von Wärme nicht möglich, und in manchen Fällen lassen sich auch Desinfektionslösungen nicht anwenden, da solche manchmal das Kunststoffmaterial angreifen könnten, aus dem der zu sterilisierende Gegenstand gefertigt ist.

In andern Fällen, beispielsweise im Fall komplex geformter Gegenstände, können sich Systeme mit Ultraviolett-Strahlung als unpraktisch erweisen, und auch die Anwendung von desinfizierenden Gasen kann die gewünschten Resultate nicht liefern.

Bedarf besteht jedoch für Mittel, die das Sterilisieren der Oberfläche kleiner Gegenstände erlauben, und die soweit wirksam sind, dass sie vollständiges Entfernen der Bakterien sicherstellen, auch falls die Bakterien spezifische Resistenz-Eigenschaften gegen bestimmte Typen von Antibiotika entwickelt haben.

Eigenen Forschungsarbeiten der Anmeldefirma wurde die Hypothese zu Grunde gelegt, dass das Anlegen elektrischer und/oder magnetischer Felder eine wirksame Oberflächen-Sterilisierung bei Gegenständen aus irgendeinem Material mit guten Resultaten und ohne Beschädigung sicherstellen könne.

Die nachfolgenden Experimente haben die Richtigkeit dieser Hypothese bestätigt und haben somit zur vorliegenden Erfindung geführt. Die Wirksamkeit der entsprechend dem erfindungsgemässen Verfahren ausgeführten Vorrichtungen wurde sodann in einer Reihe von Testversuchen auf Bakterienkulturen in Biofilmen nachgewiesen, die von Mikrobiologie-Laboratorien durchgeführt wurden.

Die vorliegenden Erfindung ist im Folgenden im Sinn nicht einschränkender Beispiele detailliert beschrieben, unter Bezugnahme auf die folgenden Abbildungen. Die zeigen in der:

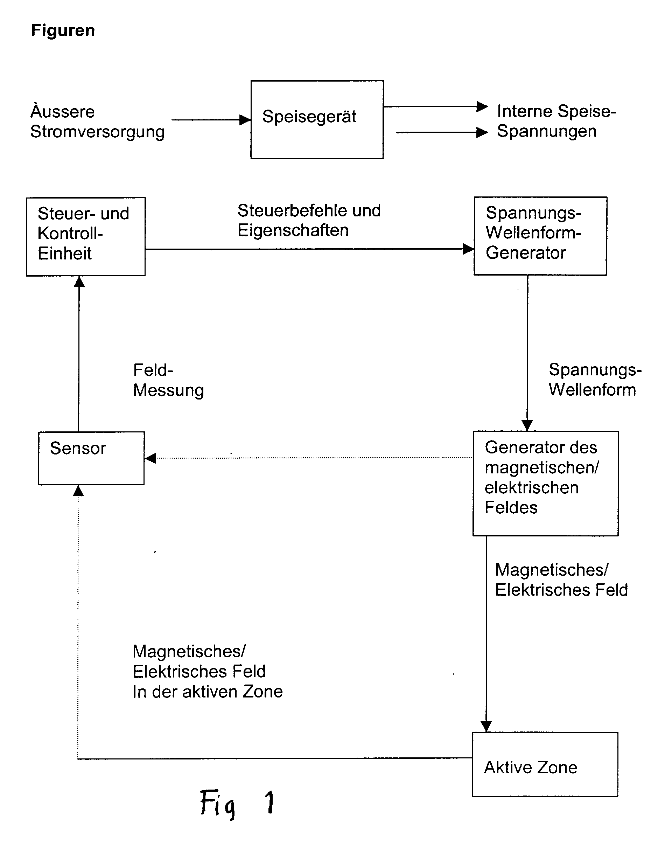

- Fig. 1: Ein Block-Schema einer Sterilisier-Einheit gemäss der vorliegenden Erfindung, mit Einrichtungen zur Erzeugung eines elektrischen und/oder magnetischen Feldes:
- Fig. 2: Eine schematische Darstellung einer Vorrichtung zur Sterilisierung gemäss der vorliegenden Erfindung;
- Fig. 3: Das elektrische Schema der Vorrichtung gemäss der Fig. 2;
- Fig. 4: Eine schematische Darstellung einer weiteren bevorzugten Ausführungsform der Vorrichtung zum Sterilisieren gemäss den Prinzipien der vorliegenden Erfindung.

Das Verfahren zum Sterilisieren gemäss der vorliegenden Erfindung sieht vor, dass der auf seiner Oberfläche zu sterilisierende Gegenstand mit einem elektrischen oder magnetischen Feld mit geeigneten Eigenschaften der Intensität und der Frequenz während einer vorausbestimmten Zeitdauer beaufschlagt wird.

Zu diesem Zweck werden gemäss der vorliegenden Erfindung Vorrichtungen zum Sterilisieren vorgesehen, die einen Raum zur Aufnahme des zu sterilisierenden Gegenstandes aufweisen sowie geeignete Mittel zum Erzeugen eines starken elektrischen und/oder magnetischen Feldes um diesen Gegenstand, mit bestimmten Frequenzeigenschaften, in solcher Weise, dass die Bakterienbesiedlung auf der Oberfläche des Gegenstandes, der diesem Feld ausgesetzt wird, entfernt wird.

Die Vorrichtung kann je nach der Art des zu sterilisierenden Gegenstandes in verschiedener Weise ausgelegt sein.

Generell umfassen die Vorrichtungen zu Ausüben der Verfahren die folgenden Funktionsgruppen:
1) Speisung
   Dies ist die elektrische/elektronische Schaltung, welche eine geeignete Speisespannung an die andern Teile der Vorrichtung abgibt, beginnend bei der verfügbaren Stromversorgungsquelle (Haushalts-Elektronetz, Batterien, Fahrzeug-Generator, usw.).
2) Spannungs-Wellenform-Generator
   Diese elektrische/elektronische Schaltung ist in der Lage, eine variable elektrische Spannung nach gewünschten Richtlinien zu erzeugen. Diese Spannung wird an den Feldgenerator des magnetischen/elektrischen Feldes geliefert.
3) Kontroll- und Steuer- Einheit
   Dies ist eine elektrische/elektronische Einheit, die als Schnittstelle der Bedienungsperson zur Kontrolle und Steuerung der andern Vorrichtungsteile dient. Die Bedienungsperson schaltet die Vorrichtung über diese Einheit ein und aus, steuert die Erzeugung des magnetischen/elektrischen Feldes, und erhält von der Einheit Angaben über den Funktionszustand. Diese Einheit steuert den Spannungs-Wellenform-Generator entsprechend den Vorgaben des Benützers, oder auf Wunsch in Funktion des Rückmeldesignals des magnetischen/elektrischen Feld-Signalübermittler (Transducers).
4) Generator des magnetischen/elektrischen Feldes
   Dies ist ein physikalisches System, das ein magnetisches/elektrisches Feld in der aktiven Zone oder im aktiven Hohlraum erzeugt, das von der Spannung abhängt, die vom Spannungs-Wellenform-Generator geliefert wird.
5) Signalübermittler (Transducer) für die Rückmeldung
   Dies ist ein Fühler (Sensor) der das in der aktiven Zone erzeugte magnetische/elektrische Feld abfühlt und ein dazu proportionales Signal an die Kontroll- und Steuereinheit zurückmelden kann.
6) Aktive Zone bzw. Aktiver Hohlraum
   Die Raumzone, in welche die allenfalls vom Biofilm betroffene Oberfläche eingebracht wird.

Die verschiedenen Komponenten der Vorrichtung, Generator, Signalübermittler, usw., sind an sich dem Fachmann auf dem Gebiet bekannt, weshalb an dieser Stelle keine genauere Beschreibung erforderlich ist.

Zum Nachweis der Wirksamkeit einer Vorrichtung dieser Art wurde ein Biofilm auf einem metallischen Stäbchen von 2 mm Durchmesser gezüchtet, das in eine Nährlösung in einem Reagenzglas eingetaucht war. Das Reagenzglas von etwa 10 mm Durchmesser wurde in einen metallischen Hohlraum eingebracht. Die beiden Pole des Spannungsgenerators wurden an das Metallstäbchen und an den metallischen Hohlraum angeschlossen, so dass ein radiales elektrisches Feld um das Metallstäbchen aufgebaut wurde, das somit auch auf den darauf gezüchteten Biofilm einwirkte.

Der verwendete Spannungsgenerator produzierte eine sinusförmige Wechselspannung mit einer Amplitude von 1000 V (Spitze zu Spitze) und mit einer Frequenz von 400 Hz. Das die Oberfläche des Metallstäbchens beaufschlagende Feld wurde auf 250 kV/m Spitze zu Spitze geschätzt.

Die erhaltenen Resultate bestätigen die Wirksamkeit des Verfahren, und danach wurden weitere Versuche mit verschiedenen Ausführungsvarianten von Vorrichtungen gemäss der vorliegenden Erfindung durchgeführt.

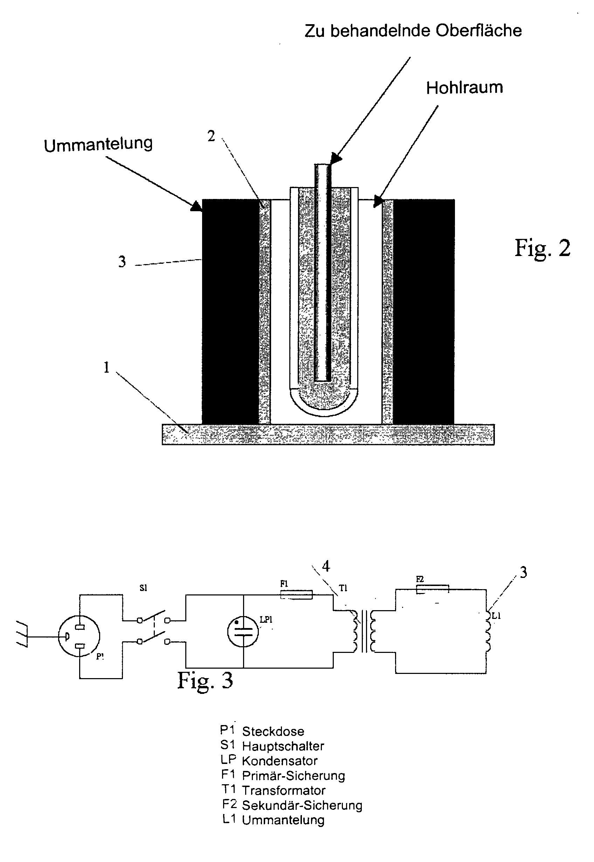

In der Fig. 2 ist ein Schnitt durch eine Vorrichtung gezeigt, die ein axiales Magnetfeld erzeugen kann, wobei das elektrische Schema dieser Vorrichtung in der Fig. 3 dargestellt ist. Diese Vorrichtung (Fig. 2) umfasst eine Unterlage 1, auf der ein Kunststoffrohr 2 aufgelötet ist, das als Träger für eine Ummantelung 3 dient.

Das erzeugte Feld verläuft längs der Achse des Rohrs sinusförmig mit einer Frequenz von 50 Hz (bzw. Netzfrequenz) und hat eine Stärke von 20 mT Spitze zu Spitze

Im Hohlraum im Innern des Rohrs 2 und der Ummantelung 3 wird somit ein Raum geschaffen, in den die zu sterilisierenden Gegenstände eingebracht werden können. Dies können Gegenstände aus nicht-ferromagnetischem Material sein, die in einer Richtung eine viel grössere Ausdehnung haben als in den andern Richtungen, wie Pinzetten, Zahnbürsten, Zahnarztspiegel oder Ähnlichem.

Die Ummantelung 3 kann mittels eines Transformators 4 direkt mit Netzstrom beliefert werden, so dass ein axiales Magnetfeld entsteht, das mit 50 Hz (bzw. Netzfrequenz) alterniert.

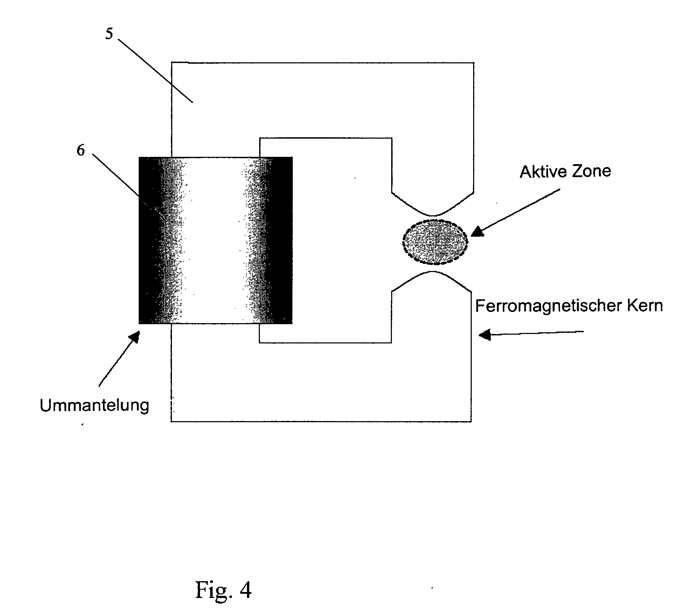

In der Fig. 4 ist hingegen eine andere Ausführungsform der Vorrichtung zum Sterilisieren gemäss der vorliegenden Erfindung dargestellt, die ein Magnetfeld in einer Raumzone zwischen zwei Polverlängerungen eines Magnetkreises erzeugt.

Dieser Magnetkreis aus einem ferromagnetischen "C"-förmigen Kern besteht, der mit 5 bezeichnet ist, auf dem eine Ummantelung 6 angebracht ist.

Der Hohlraum in der "C"-Form stellt die aktive Zone dar, und das sinusförmig mit der Netzfrequenz von 50 Hz alternierende Magnetfeld weist eine Amplitude von 250-300 mT Spitze zu Spitze auf.

Das Vorhandensein eines ferromagnetischen Kerns erlaubt erhöhte Feldintensitäten zu erreichen, auch wenn die aktive Zone keine grossen Abmessungen aufweisen kann.

Diese Auslegung erweist sich als besonders geeignet, um kleine Oberflächen mit sehr intensiven Feldern zu behandeln, man denke etwa an die Desinfektion von Nadeln oder Ähnlichem.

Die ausgeführten Experimente haben die Wirksamkeit des Systems bestätigt und haben auch nachgewiesen, dass je nach der Art des zu behandelnden Gegenstandes, dem Material aus dem er besteht, seiner Form und seinen Abmessungen, bessere Resultate oder kürzere Behandlungszeiten erreicht werden können, wenn die Eigenschaften des magnetischen oder elektrischen Feldes in geeigneter Weise variiert werden.

Solche Vorrichtungen, die im wesentlichen gleich aufgebaut sein können wie die unter Bezugnahme auf die vorhergehenden Beispiele in den Abbildungen gezeigten, verwenden zum Erzeugen des Magnetfeldes eine Ummantelung auf einem für Felder mit höherer Frequenz geeigneten Ferritkern, der durch einen elektronischen Schaltkreis gespeist wird, der mit variabler Frequenz alterniert.

Die Bedienungsperson stellt mittels des Displays die Frequenz und die Amplitude des Wechselfeldes ein, während die Elektronik bekannter Art für die Erzeugung eines entsprechenden Signals sorgt, das über die Zeit variiert werden kann.

Die Verfahren und die Vorrichtungen gemäss der vorliegenden Erfindung erwiesen sich als wirksam, wenn auch die Mechanismen, die zum Erreichen dieser Resultate führen, noch nicht genauer bekannt sind.

Man kann annehmen, dass die während der Behandlung auf die Bakterien übertragene Energie deren Vernichtung hervorruft, vermutlich durch Wärme.

Auf jeden Fall haben die durch Mikrobiologie-Labors ausgeführten Versuche mit Bakterienkulturen in Biofilm-Form die Wirksamkeit des Systems bestätigt.

Der Fachmann auf dem Gebiet wird im Rahmen der erfinderischen Idee verschiedene Ausführungsformen der Vorrichtungen aufzeigen können, die jedoch alle im Rahmen der vorliegenden Erkenntnisse liegen müssen.

### Beispiel - Mikrobiologischer Testversuch

### Verwendete Bakterienstämme:

*Pseudmonas aeruginosa* (Klinisch isolierter Stamm, produziert Biofilm), *Staphylococcus epidermidis* (Klinisch isolierter Stamm, produziert Biofilm), *Staphylococcus aureus* ATCC 259271 (produziert Biofilm).

Substrate und Pufferlösungen für die Zucht der Stämme in der sesshaften Form und für die Kontrolle dieser Stämme in planktonischer (freischwimmender) Form:
*Substrat:* Brain Hearth Infusion (BHI) (Oxoid)
*Puffer:* PBS (Sigma Produkte)
NaCl 8 g
Na₂HPO₄ 1.44 g
KH₂ PO₄ 0.24 g
KCl 0.2 g

Vor jedem Versuch wurde jeder Bakterienstamm nochmals überprüft, mittels Aufzucht auf festem Substrat (BHI) und Arten-Identifikation mittels automatischer Systeme (API Systeme).

Verwendet wurden Metallstäbchen und Kügelchen aus Polystyren (Polystyrol) (Produkt der Bead Corporation , USA) als künstliche Unterlage für die Aufzucht von Biofilm. Die Bakterienstämme wurden in bekannten Mengen (10⁶ cfu) in der Lösung (BHI) in Gegenwart der vorgewählten Unterlage eingeimpft. Die Bakterien und die Unterlagen wurden sodann für mindestens 5 Tage bei 37°C in der Kulturlösung belassen: In dieser Weise können sich die Bakterien auf den Unterlagen ansiedeln und ihre Kolonienbildung bis zum reifen Biofilm führen.

Nach 5 Tagen wurde jede der Unterlagen der folgenden Behandlung unterworfen:
- Mindestens zweimaliges Waschen in steriler PBS (wobei der Waschvorgang eine Wirbelbehandlung von mindestens einer Minute Dauer umfasst), mit dem Zweck, die freischwimmenden (planktonischen) Bakterien vollständig zu entfernen;
- Die solchermassen behandelte Unterlage wurde dem vorgewählten Behandlungsvorgang unterzogen;
- Nach der Behandlung wurde jede der Unterlagen in PBS eingetaucht und während mindestens 2 Minuten einer Ultraschallbehandlung im Ultraschallgerät Sorvall unterzogen.
- In solcher Weise wurden aus dem Biofilm die Bakterienzellen in die umgebende Flüssigkeit (PBS) befreit; letztere kann dazu verwendet werden, eine Quantifizierung der Bakterien vorzunehmen mittels Zählung der Kolonien auf festem Substrat (Siehe später). Die Ultraschallbehandlung wurde bei jeder Unterlage dreimal wiederholt. Nach jeder Ultraschallbehandlung wurden 100 Mikroliter PBS mit den aus dem Biofilm herausgelösten Bakterienzellen auf Petrischalen mit festem BHI aufgebracht, um die Anzahl der Bakterien festzustellen, welche die Behandlung überlebt hatten. Die Zahl der überlebenden Bakterien wurde ermittelt, indem die Zahl der auf dem festen Substrat nach 24 h Inkubationszeit bei 37°C entstandenen Kolonien ausgezählt wurde. Darüber hinaus wurde zur besseren Kontrolle jede der den drei Ultraschabehandlungen unterworfenen Unterlagen in eine Nährlösung (BHI) bei 37°C für 24 h eingelegt, um allfällig verbliebenen, noch lebenden und durch die Ultraschallbehandlung noch nicht abgelösten Biofilm festzustellen.
- Diese Behandlung jeden Tag während 5 Tagen wiederholt (beim letzten Versuch während 6 Tagen).

### Versuchsresultate:

### Versuche mit elektrischen Feldern

| | 1. Tag | | 2. Tag | | 3. Tag | | 4. Tag | | 5. Tag | | 6. Tag | | 7. Tag | |
|---|---|---|---|---|---|---|---|---|---|---|---|---|---|---|
| Min/ Volt | 40' 1000 | 2' 12000 | 40' 1000 | 2' 12000 | 40' 1000 | 2' 12000 | 40' 1000 | 2' 12000 | 40' 1000 | 2' 12000 | 40' 1000 | 2' 12000 | 40' 1000 | 2' 12000 |
| *Staph. epiderm.* | +++ | +++ | 50% | 50% | - | - | - | - | - | - | - | - | - | - |
| *Staph. aureus* | +++ | +++ | 50% 80% | 50% | - | - | - | - | - | - | - | - | - | - |
| Pseud. aerugin. | +++ | +++ | +++ | +++ | +++ | ? | +++ | +++ | ++- | ++- | ++- | ++- | ++- | +1- |

### Versuch mit einer ersten Vorrichtung mit Magnetfeld (Metallstäbchen im Zentrum eingetaucht)

### Magnetfeld, Gram positive und Gram-negative Stämme

| | 1. Tag | 2. Tag | 3. Tag | 4. Tag | 5. Tag | 6. Tag | Kontrolle |
|---|---|---|---|---|---|---|---|
| Pseudomonas *aeruginosa* | +++ | ++ | ++- | +-- | ++- | ++- | +++ |
| *Staphyloc. aureus* | ++- | ++- | --+ | --+ | +-- | +-- | +++ |
| *Staphyloc. epidermidis* | +-- | --- | --- | --- | --- | --- | +++ |

### Versuch mit der zweiten Vorrichtung mit Magnetfeld (mit eingetauchten Kügelchen)

| | 1. Tag | 2. Tag | 3. Tag | 4. Tag | 5. Tag | 6. Tag | Stamm-Kontrolle | Kügelchen-Kontr. PBS vor Versuch |
|---|---|---|---|---|---|---|---|---|
| *Pseudomonas aeruginosa* | +++ | +++ | +++ | +++ | +++ | +++ | +++ | +++ |
| *Staphyloc. aureus* | +++ | +++ | +++ | +++ | +++ | +++ | +++ | +++ |
| *Staphyloc. epidermidis* | - | | | | | | ++ | ++ |
| **Legende:** +++ 1200 Kolonien pro Petrischale | | | | | | | | |
| ++ 600 Kolonien pro Petrischale | | | | | | | | |
| + 300 Kolonien pro Petrischale | | | | | | | | |
| +- 150 Kolonien pro Petrischale | | | | | | | | |
| -+ 100 Kolonien oder weniger pro Petrischale | | | | | | | | |
| - Keine Zunahme auch in der Kontroll-Nährlösung der Unterlage nach Ultraschallbehandlung | | | | | | | | |

## Patentansprüche

1. Verfahren zum Sterilisieren der Oberflächen von Gegenständen zwecks Entfernung von Bakterienfilmen
**dadurch gekennzeichnet, dass**
die Oberfläche des Gegenstandes mit einem elektrischen und/oder magnetischen Feld beaufschlagt wird, um die Vernichtung der Bakterien hervorzurufen.

2. Verfahren gemäss dem Anspruch 1,
**dadurch gekennzeichnet, dass**
die Oberfläche des Gegenstandes mit einem über die Zeit variablen elektrischen und/oder magnetischen Feld beaufschlagt wird.

3. Vorrichtung zur Ausübung des Verfahrens gemäss den Ansprüchen 1 oder 2, **dadurch gekennzeichnet, dass** folgende Einheiten vorgesehen sind:
• Ein metallischer Behälter mit einem Aufnahmeraum, in den ein zu behandelnder Metallgegenstand eingebracht werden kann.
• Ein Spannungsgenerator, und
• Mittel zum Verbinden der Pole des genannten Spannungsgenerators mit dem Metallgegenstand und mit dem metallischen Behälter, in dem der Gegenstand untergebracht ist.

4. Vorrichtung zur Ausübung des Verfahrens gemäss den Ansprüchen 1 oder 2, **dadurch gekennzeichnet, dass** folgende Einheiten vorgesehen sind:
• Ein Körper (2), der einen Hohlraum aufweist, der als Aufnahmeraum dienen kann, in den ein zu sterilisierender Gegenstand eingebracht werden kann;
• Eine um den genannten Körper angebrachte Ummantelung (3);
• Mittel (4) zum Zirkulieren eines Wechselstroms in der genannten Ummantelung, um ein Magnetfeld im Innern des genannten Hohlraums, der zur Aufnahme des zu sterilisierenden Gegenstandes dient, zu erzeugen.

5. Vorrichtung zur Ausübung des Verfahrens gemäss den Ansprüchen 1 oder 2, **dadurch gekennzeichnet, dass** folgende Einheiten vorgesehen sind:
• Ein Kern aus ferromagnetischem Material, der zwei einander gegenüberliegende Polenden aufweist, wobei der Zwischenraum zwischen den genannten Polenden die aktive Zone bildet, in die der zu sterilisierende Gegenstand eingebracht werden kann;
• Eine auf dem genannten Kern angebrachte Ummantelung;
• Mittel (4) zum Zirkulieren eines Wechselstroms in der genannten Ummantelung, um ein Magnetfeld im Innern der genannten aktiven Zone, die zur Aufnahme des zu sterilisierenden Gegenstandes dient, zu erzeugen.

6. Vorrichtungen zum Ausüben des Verfahrens gemäss den Ansprüchen 1 oder 2, gemäss den Beschreibungen und Abbildungen.
